# FASCICULE DE BREVET EUROPEEN

(11) **EP 0 723 772 B1**
(45) Date de publication et mention de la délivrance du brevet: **04.03.1998**
(21) Numéro de dépôt: 96400058.2
(22) Date de dépôt: 10.01.1996
(51) Int. Cl.: A61K 7/09

(54) **Composition réductrice comprenant un acide aminé basique et un polymère cationique**
Reduzierende Zusammensetzung auf Basis einer basischen Aminosäure und eines Kationischen Polymers
Reducing composition bared on a basic amino acid and a cationic polymer

(30) Priorité: 30.01.1995 FR 9501038
(43) Date de publication de la demande: 31.07.1996
(73) Titulaire: L'OREAL, 75008 Paris (FR)
(72) Inventeur: de Labbey, Arnaud, F-93600 Aulnay-sous-Bois (FR); Pataut, Françoise, F-75017 Paris (FR)
(74) Mandataire: Tezier Herman, Béatrice

(56) Documents cités:
- EP-A- 0 235 783
- EP-A- 0 461 526
- FR-A- 2 707 486
- GB-A- 2 066 310
- US-A- 5 332 570
- PATENT ABSTRACTS OF JAPAN vol. 3, no. 109 (C-058) & JP-A-54 086635 (AJINOMOTO CO INC.), 10 Juillet 1979,

## Description

La présente invention concerne une nouvelle composition cosmétique pour le premier temps d'une opération de déformation permanente des matières kératiniques, en particulier des cheveux, comprenant un acide aminé basique, en tant qu'agent alcalinisant, et des polymères cationiques.

Elle concerne également un procédé de traitement des matières kératiniques, en particulier des cheveux, en vue d'obtenir une déformation permanente de ces dernières, en particulier sous la forme de cheveux permanentés.

On sait que la technique la plus usuelle pour obtenir une déformation permanente des cheveux, consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

Les compositions réductrices utilisables pour la mise en oeuvre de la première étape d'une opération de permanente contiennent généralement, à titre d'agents réducteurs, des sulfites, des bisulfites ou, de préférence, des thiols. Parmi ces derniers, ceux couramment utilisés sont la cystéïne et ses divers dérivés, la cystéamine et ses dérivés, l'acide thiolactique, l'acide thioglycolique ainsi que ses esters, notamment le monothioglycolate de glycérol, et le thioglycérol.

A cet égard, et bien que possédant une odeur désagréable, l'acide thioglycolique est particulièrement efficace, et constitue ainsi le composé de référence en permanente pour réduire les liaisons disulfures de la kératine ; la cystéïne, quant à elle, produit une odeur beaucoup plus faible que celle de l'acide thioglycolique ou du monothioglycolate de glycérol, mais le degré de frisure obtenu est malheureusement inférieur et loin d'être totalement satisfaisant.

Ces agents réducteurs ayant un effet détériorant sur les cheveux, il a été préconisé de les associer à des polymères cationiques.

Cependant, ces compositions présentent globalement une odeur forte et piquante, ce qui est incommodant pour l'utilisateur et pour son entourage, plus particulièrement dans les salons de coiffure où la fréquence de ce type de traitements est particulièrement élevée. L'odeur désagréable de ces compositions est due aux agents réducteurs et aux agents alcalinisants, les agents alcalinisants généralement utilisés étant soit l'ammoniaque, soit la monoéthanolamine.

De plus, l'utilisation de ces compositions sur des cheveux sensibilisés pour obtenir une déformation permanente de ces cheveux ne donne pas entière satisfaction, tant au niveau du résultat de la frisure, qu'au niveau de la qualité des cheveux.

Il est connu du document EP-A-461 526 des compositions cosmétiques pour le premier temps d'une opération de déformation permanente des matières kératiniques comprenant comme agent réducteur un éther de thioglycéryl particulier et comprenant un agent tampon et un polymère.

Un autre problème des techniques de permanentes connues à ce jour est que leur application répétée sur les cheveux induit à la longue une modification importante du comportement de ces derniers, en particulier au niveau de leur aptitude à être par la suite correctement colorés. Ainsi, on observe tout d'abord que, sur des cheveux qui ont subi quelques opérations de permanente, la coloration sera beaucoup plus prononcée que celle obtenue sur les mêmes cheveux mais non permanentés; ceci pose donc un problème dans tous les cas où l'opération de coloration est conduite sur une chevelure à l'origine permanentée mais qui, entre temps, a aussi repoussée (mauvaise unisson entre les cheveux d'origine permanentés et les cheveux de repousse non permanentés). On observe par ailleurs que la coloration devient très difficile, voire impossible, si la chevelure à colorer a subi auparavant de nombreuses opérations de permanentes.

D'autre part, lorsque l'agent alcalinisant présent pour tamponner le pH de la composition réductrice est choisi parmi des produits carbonatés, comme par exemple le gaz carbonique, les carbonates ou bicarbonates d'alcalins ou d'ammonium, les carbonates organiques tels que notamment le carbonate de guanidine, il s'avère malheureusement que l'application répétée des opérations de mises en forme/déformation permanentes au moyen de ces compositions réductrices carbonatées associées à des compositions oxydantes à base d'eau oxygénée, entraîne à la longue une altération progressive et marquée de la qualité du cheveu, en particulier au niveau de la douceur des fibres qui ont tendance à devenir de plus en plus rêches.

La présente invention a notamment pour but de résoudre les problèmes ci-dessus.

Plus précisément encore, la présente invention a pour but de proposer une composition telle que ci-dessus qui permet de limiter, voire supprimer, la dégradation mécanique du cheveu, après répétition du traitement.

Elle a aussi pour but de proposer une composition telle que ci-dessus qui soit, globalement, peu odorante d'une part, et peu irritante pour la peau et/ou le cuir chevelu d'autre part.

Elle a également pour but de proposer un nouvelle composition cosmétique pour le premier temps d'une opération de déformation permanente des cheveux permettant d'obtenir un rendement, une nervosité et une beauté de frisure satisfaisants.

Enfin, la présente invention a pour but de proposer une composition cosmétique pour le premier temps d'une opération de déformation permanente des cheveux permettant d'améliorer les qualités de colorations ultérieures.

Or, il a été trouvé par la demanderesse que ces buts, et d'autres, pouvaient être atteints avec succès en procédant à une sélection convenable des composés de la composition dite réductrice. Cette découverte est à la base de la présente invention.

Ainsi, il est maintenant proposé selon la présente invention une nouvelle composition cosmétique pour le premier temps d'une opération de déformation permanente des matières kératiniques, en particulier des cheveux, consistant à réduire les liaisons disulfures de la kératine, dite composition réductrice, caractérisée par le fait qu'elle comprend au moins un agent actif approprié pour la réduction des liaisons disulfures de la kératine, au moins un agent alcalinisant choisi parmi l'ornithine, la lysine et l'arginine, et au moins un polymère cationique comportant des groupements amine primaire, secondaire, tertiaire ou ammonium quaternaire dans la chaîne principale.

La présente invention concerne également un procédé de traitement des matières kératiniques, en particulier des cheveux, en vue d'obtenir une déformation permanente de ces dernières, en particulier sous la forme de cheveux permanentés, ledit procédé étant caractérisé par le fait qu'il comporte les étapes suivantes : (i) on applique sur la matière kératinique à traiter une composition telle que définie ci-dessus, les moyens (rouleaux) nécessaires à la mise sous tension mécanique de la matière kératinique étant mis en oeuvre avant, pendant ou après ladite application, (ii) puis on rince la matière kératinique ainsi traitée, (iii) on applique sur la matière kératinique ainsi rincée une composition oxydante, la matière kératinique traitée étant séparée des moyens de mise sous tension utilisés à l'étape (i) avant ou après ladite application de la composition oxydante, (iv) et enfin on rince à nouveau la matière kératinique.

Le procédé selon l'invention convient particulièrement bien à l'obtention d'une chevelure permanentée.

Appliquée sur une chevelure saine, et même de manière répétitive, la composition selon l'invention présente pour avantages principaux, entre autres, de conduire, et ceci sans dégagement d'odeurs désagréables d'une part et d'une façon non irritante pour la peau et/ou le cuir chevelu d'autre part, à des cheveux moins abîmés, résistants mécaniquement, et présentant de belles frisures.

Mais d'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Parmi les agents actifs appropriés pour la réduction des liaisons disulfures de la kératine, on peut citer des sulfites, des bisulfites ou, de préférence, des thiols. Parmi ces derniers, ceux préférentiellement utilisés sont la cystéïne et ses divers dérivés, la cystéamine et ses dérivés, l'acide 3-mercaptopropionique, l'acide thiolactique, l'acide thioglycolique ainsi que leurs esters ou leurs sels, notamment le monothioglycolate de glycérol, et le thioglycérol.

Ces agents actifs peuvent être utilisés seuls ou en mélange.

Parmi les agents alcalinisants utilisés dans la présente invention, on préfère utiliser l'arginine.

L'agent alcalinisant choisi parmi l'ornithine, la lysine et l'arginine est donc utilisé pour atteindre le pH désiré, le pH devant être généralement compris entre 5 et 11,5.

Il est bien entendu possible d'utiliser ces agents alcalinisants seuls ou en mélange. Ils peuvent également être présents avec d'autres agents alcalinisants, tels que l'ammoniaque, la monoéthanolamine ou des produits carbonatés. Ces autres agents alcalinisants sont de préférence présents en des quantités ne leur permettant pas de donner aux compositions les contenant les inconvénients ci-dessus mentionnés, tels que les mauvaises odeurs.

En particulier, ces autres agents alcalinisants sont utilisés pour neutraliser les agents réducteurs à caractère acide.

Lorsque l'on utilise l'acide thioglycolique, l'acide thiolactique, l'acide 3-mercaptopropionique, la cystéine ou la cystéamine, ou l'un de leurs sels ou de leurs dérivés, à titre d'agent réducteur, le pH de l'ensemble de la composition selon l'invention est de préférence compris entre 6,5 et 11,5 et encore plus préférentiellement entre 7 et 10.

Lorsque l'on utilise les esters de l'acide thioglycolique ou l'acide thiolactique ou l'acide 3-mercaptopropionique, à titre d'agent réducteur, le pH de l'ensemble de la composition selon l'invention est de préférence compris entre 5 et 11 et encore plus préférentiellement entre 6 et 9,5.

Les polymères cationiques utilisés dans la présente invention comportent des groupements amine primaire, secondaire, tertiaire ou ammonium quaternaire dans la chaîne principale. Ils on en général un poids moléculaire supérieur à 500, de préférence supérieur à 1000.

Les polymères cationiques préférentiellement utilisés dans la présente invention sont choisis parmi :
(1) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(2) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.
   De tels polymères sont notamment décrits dans les brevets français 2.252.840 et 2.368.508 ;
(3) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle.
   De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(4) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1.
   De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(5) les cyclohomopolymères de méthyl diallyl amine ou de diméthyl diallyl ammonium tels que les homopolymères comportant comme constituant principal de la chaîne des motifs répondant aux formules (I) ou (II) : 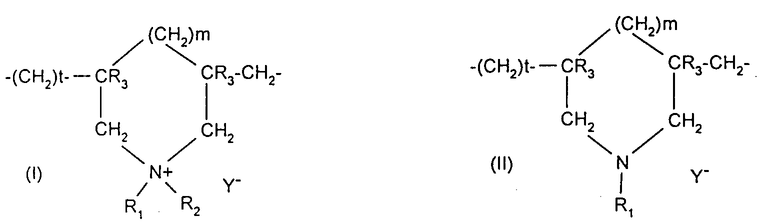 formules dans lesquelles m et t sont égaux à 0 ou 1, la somme m + t étant égale à 1 ; R3 désigne un atome d'hydrogène ou un radical méthyle ; R1 et R2, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R1 et R2 peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate.
   Parmi les polymères définis ci-dessus, on peut citer plus particulièrement l'homopolymère de chlorure de diméthyldiallylammonium vendu sous la dénomination "Merquat 100" par la société Merck.
   Ces polymères sont notamment décrits dans le brevet français 2.080.759 et dans son certificat d'addition 2.190.406.
(6) le polymère de diammonium quaternaire contenant des motifs récurants répondant à la formule : 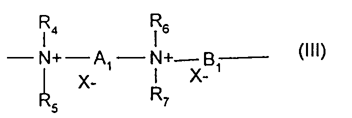 formule (III) dans laquelle :
   R4, R5, R6 et R7, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R4, R5, R6 et R7, ensemble ou séparément, constituent avec les atomes d'azote auxquels ils sont rattachés des hétérocycles contenant éventuellement un second hétéroatome autre que l'azote ou bien R4, R5, R6 et R7 représentent un radical alkyle en C1-C6 linéaire ou ramifié substitué par un groupement nitrile, ester, acyle, amide ou -CO-O-R8-D ou -CO-NH-R8-D où R8 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R4 et R6 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2- -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent -CH2-CH2-S-S-CH2-CH2-;
   d) un groupement uréylène de formule : -NH-CO-NH- ;

   De préférence, X⁻ est un anion tel que le chlorure ou le bromure.
   Ces polymères ont une masse moléculaire généralement comprise entre 1000 et 100000.
   Des polymères de ce type sont notamment décrits dans les brevets français 2.320.330, 2.270.846, 2.316.271, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(7) les polymères de polyammonium quaternaires constitués de motifs de formule (IV) : 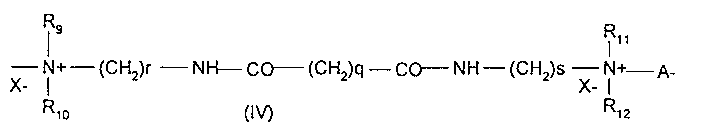 formule dans laquelle :
   R9, R10, R11 et R12, identiques ou différents, représentent un atome d'hydrogène ou un radical méthyle, éthyle, propyle, β-hydroxyéthyle, β-hydroxypropyle ou -CH2CH2(OCH2CH2)pOH,
   où p est égal à 0 ou à un nombre entier compris entre 1 et 6, sous réserve que R9, R10, R11 et R12 ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH2-CH2-O-CH2-CH2-.

De tels composés sont notamment décrits dans la demande de brevet EP-A-122 324.

On peut par exemple citer parmi ceux-ci, les produits "Mirapol A 15", "Mirapol AD1", "Mirapol AZ1" et Mirapol 175" vendus par la socitété Miranol.

Parmi ces polymères cationiques, on utilise de préférence les polymères choisis parmi le Merquat 100 et le composé de formule (III) dans laquelle R4, R5, R6 et R7 représentent le radical méthyle, A1 représente le radical de formule -(CH2)3- et B1 représente le radical de formule -(CH2)6- et X⁻ représente l'anion chlorure (nommé ultérieurement Mexomère PO).

Selon un mode de réalisation préféré, la composition réductrice contient également un agent tensioactif de type non-ionique, anionique, cationique ou amphotère couramment utilisé dans les compositions réductrices de permanentes et, parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, mais de préférence comprise entre 0,5 et 10 % en poids par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature anionique, non-ionique ou amphotère.

On peut utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonylalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy- (stéaroxydiméthicone), un copolymère polydiméthylsiloxane-dialkylammonium acétate ou un copolymère polydiméthyl-siloxane polyalkylbétaïne décrits dans la demande de brevet britannique n° 2 197 352, des polysiloxanes organo modifés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des cires, des agents de gonflement et de pénétration ou permettant de renforcer l'efficacité du réducteur tels que le diméthylisosorbitol, l'urée et ses dérivés, la pyrrolidone, les N-alkyl-pyrrolidones, la thiamorpholinone, les alkyléthers d'alkylèneglycol ou de dialkylèneglycol tels que par exemple le monométhyléther de propylène glycol, le monométhyléther de dipropylène glycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de mise en suspension, des agents séquestrants, des agents opacifiants, des colorants, des filtres solaires, ainsi que des parfums et des conservateurs.

Enfin, les compositions peuvent être également sous forme dite "auto-neutralisante" ou encore "auto-régulée" et dans ce cas, les agents réducteurs sont associés à au moins un disulfure connu pour son utilisation dans une composition réductrice pour permanente auto-neutralisante.

Parmi de tels disulfures connus, on peut notamment mentionner l'acide dithiodiglycolique, le dithioglycérol, la cystamine, la N, N'-diacétyl-cystamine, la cystine, la pantéthine, et les disulfures des N-(mercapto-alkyl)ω-hydroxyalkylamides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, et les disulfures des alkylaminomercaptoalkylamides décrits dans la demande de brevet EP-A-514 282. Ces disulfures sont généralement présents dans un rapport molaire de 0,5 à 2,5, et de préférence de 1 à 2, par rapport à l'agent réducteur (voir brevet US 3 768 490).

Dans les compositions réductrices de permanente utilisables dans le cadre de l'invention, les agents réducteurs mentionnés ci-avant sont généralement présents à une concentration qui peut être comprise entre 1 et 20 % en poids, et de préférence entre 5 et 15 % en poids par rapport au poids total de la composition réductrice.

Dans les compositions réductrices de permanente utilisables dans le cadre de l'invention, les polymères cationiques comportant des groupements amine primaire, secondaire, tertiaire ou ammonium quaternaire dans la chaîne principale tels que définis ci-dessus sont généralement présents à une concentration comprise entre 0,1 et 5 % en poids, et de préférence entre 0,5 et 3 % en poids par rapport au poids total de la composition réductrice.

Dans les compositions réductrices de permanente utilisables dans le cadre de l'invention, l'agent alcalinisant tel que défini ci-dessus est donc présent à une concentration nécessaire pour ajuster le pH de cette composition, cette concentration étant généralement comprise entre 0,01 et 20 % en poids, et de préférence entre 0,1 et 18 % en poids par rapport au poids total de la composition réductrice.

La composition réductrice peut se présenter sous la forme d'une lotion, épaissie ou non, d'une crème, d'un gel, ou de toute autre forme appropriée.

Selon une variante de l'invention, la composition réductrice selon l'invention est conditionnée de manière à ce qu'elle contient conjointement les composés précédemment décrits, la composition selon l'invention étant contenue dans un dispositif à un seul compartiment,.

Selon une autre variante de l'invention, la composition réductrice selon l'invention est contenue dans un dispositif à au moins deux compartiments, le mélange des constituants de la composition réductrice étant réalisé au moment de l'emploi. Ainsi, plus particulièrement, dans un premier compartiment, se trouve au moins un agent actif approprié pour la réduction des liaisons disulfures de la kératine, et dans le deuxième compartiment, se trouvent au moins un agent alcalinisant choisi parmi l'ornithine, la lysine et l'arginine et événuellement les parfums. Les autres composés, et plus particulièrement le polymère cationique comportant des groupements amine primaire, secondaire, tertiaire ou ammonium quaternaire dans la chaîne principale, se trouvent soit dans un de ces deux compartiments, de préférence dans le deuxième compartiment, soit dans d'autres compartiments. Cette variante de l'invention permet de présenter une composition réductrice selon l'invention très peu odorante.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, de l'isopropanol, du propylène glycol ou encore du glycérol à une concentration maximale de 20 % par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Conformément à la première étape du procédé selon la présente invention (étape (i)), la composition selon l'invention est alors appliquée sur les cheveux à traiter, lesquels auront été de préférence préalablement mouillés.

Cette application peut être réalisée avant, pendant ou après l'habituelle étape de mise sous tension des cheveux sous une forme correspondant à la forme finale désirée pour ces derniers (boucles par exemple), cette étape pouvant elle-même être mise en oeuvre par tout moyen, mécanique notamment, approprié et connu en soi pour maintenir sous tension des cheveux, tels que par exemple rouleaux, bigoudis et analogues.

Cette application comprend un temps de pose pour laisser agir la composition selon l'invention, qui est généralement compris entre 5 et 60 minutes, de préférence entre 5 et 30 minutes.

Dans une deuxième étape du procédé selon l'invention (étape (ii)), les cheveux imprégnés de composition réductrice sont ensuite rincés soigneusement, généralement à l'eau.

Selon une troisième étape essentielle du procédé de traitement selon l'invention (étape (iii)), on applique sur les cheveux ainsi rincés une composition oxydante permettant de reformer les liaisons disulfures de la kératine (étape de fixation).

La composition oxydante est du type couramment utilisée et contient comme agent oxydant par exemple l'eau oxygénée, un bromate alcalin, un persel ou un mélange de bromate alcalin et d'un persel.

La concentration en eau oxygénée peut varier de 1 à 10 volumes, mais est de préférence de 8 volumes, la concentration en bromate alcalin est de 1 à 12 % et celle en persel de 0,1 et 15 % en poids par rapport au poids total de la composition oxydante.

Le pH de la composition oxydante peut varier entre 2 et 8, mais de préférence entre 3 et 6.

L'eau oxygénée peut être stabilisée par exemple par la phénacétine, l'acétanilide, les phosphates mono et trisodiques ou par le sulfate d'hydroxy-8 quinoléine.

La composition oxydante peut également contenir des agents alcalinisants ou acidifiants, des agents conservateurs, des agents séquestrants, des opacifiants et éventuellemnt un polymère cationique tel que défini ci-dessus pour la composition réductrice.

On peut retirer de la chevelure les moyens mécaniques (rouleaux, bigoudis et analogues) qui maintenaient sous tension et dans la forme désirée les cheveux tout au long du traitement avant ou après l'étape de fixation.

Enfin, dans la dernière étape du procédé selon l'invention (étape(iv)), on rince abondamment les cheveux ainsi traités.

La présente invention a également pour objet un procédé de défrisage ou de décrêpage des cheveux dans lequel on applique sur les cheveux une composition réductrice selon l'invention puis l'on soumet les cheveux à une déformation mécanique permettant de les fixer dans leur nouvelle forme, par une opération de lissage des cheveux avec le dos ou les dents d'un peigne ou à la main. Pendant le temps de pose de 5 à 60 minutes, en particulier de 5 à 30 minutes, on procède alors à un ou plusieurs nouveaux lissages puis on rince soigneusement et on applique une composition oxydante ou fixatrice telle que définie ci-dessus, que l'on laisse agir pendant environ 2 à 10 minutes puis on rince abondamment les cheveux.

Des exemples concrets illustrant l'invention vont maintenant être donnés. Dans ces exemples, MA signifie matière active.

### EXEMPLE 1

On prépare selon l'invention une composition réductrice de déformation permanente des cheveux en procédant au mélange des ingrédients suivants :

| | |
|---|---|
| - Acide thioglycolique | 9,2 g |
| - Arginine | 2,0 g |
| - Ammoniaque à 20% de NH3 | 9,3 g (1,86g MA) |
| - Mexomère PO | 1,0 g MA |
| - Carbonate d'ammonium | 4,5 g |
| - Cocoylamidopropyl bétaïne/monolaurate de glycérol (25/5) en solution aqueuse à 30% | 1,3 g (0,39g MA) |
| - Parfum | 0,4 g |
| - Peptisant | 0,8 g |
| - Séquestrant | 0,4 g |
| - Eau déminéralisée | qsp 100 g |
| | pH 8,3 |

Cette composition est appliquée sur des cheveux mouillés préalablement enroulés sur des rouleaux de mise en plis. Après avoir laissé agir la composition pendant environ 15 min., on rince abondamment à l'eau puis on applique la composition oxydante suivante :

| | |
|---|---|
| - Eau oxygénée | qsp 8 volumes |
| pH 3 | |

On laisse agir la composition oxydante pendant environ 5 minutes, puis on rince abondamment la chevelure à l'eau et on enlève les rouleaux.

Après séchage sous casque, les cheveux présentent de belles boucles.

### EXEMPLE 2

On prépare selon l'invention une composition réductrice de déformation permanente des cheveux en procédant au mélange des ingrédients suivants :

| | |
|---|---|
| - Acide thioglycolique | 6,2 g |
| - Thioglycolate d'ammonium | 2,9 g |
| - Arginine | 15 g |
| - Mexomère PO | 1,0 g MA |
| - Cocoylamidopropyl bétaïne/monolaurate de glycérol (25/5) en solution aqueuse à 30% | 1,3 g (0,39g MA) |
| - Parfum | 0,5 g |
| - Peptisant | 1 g |
| - Séquestrant | 0,4 g |
| - Eau déminéralisée | qsp 100 g |
| | pH 8,3 |

Cette composition est appliquée sur des cheveux mouillés préalablement enroulés sur des rouleaux de mise en plis. Après avoir laissé agir la composition pendant environ 15 min., on rince abondamment à l'eau puis on applique la composition oxydante suivante :

| | |
|---|---|
| - Eau oxygénée | qsp 8 volumes |
| pH 3 | |

On laisse agir la composition oxydante pendant environ 5 minutes, puis on rince abondamment la chevelure à l'eau et on enlève les rouleaux.

Après séchage sous casque, les cheveux présentent de belles boucles.

### EXEMPLE 3

On réalise la même composition que dans l'exemple 2, mais conditionnée dans un dispositif à deux compartiments.

### Compartiment A :

| | |
|---|---|
| - Acide thioglycolique | 6,2 g |
| - Thioglycolate d'ammonium | 2,9 g |
| - Séquestrant | 0,4 g |
| - Eau déminéralisée Compartiment B : | qsp 30 g |
| - Arginine | 15 g |
| - Mexomère PO | 1,0 g MA |
| - Cocoylamidopropyl bétaïne/monolaurate de glycérol (25/5) en solution aqueuse à 30% | 1,3 g (0,39g MA) |
| - Parfum | 0,5 g |
| - Peptisant | 1 g |
| - Eau déminéralisée | qsp 70 g |

Ce type de formulation permet, notamment grâce à une meilleure conservation du parfum (pas en présence des thiols), de présenter une odeur nettement améliorée.

Une fois les constituants de cette composition mélangés, on applique cette composition sur des cheveux mouillés préalablement enroulés sur des rouleaux de mise en plis. Après avoir laissé agir la composition pendant environ 15 min., on rince abondamment à l'eau puis on applique la composition oxydante suivante:

| | |
|---|---|
| - Eau oxygénée | qsp 8 volumes |
| pH 3 | |

On laisse agir la composition oxydante pendant environ 5 minutes, puis on rince abondamment la chevelure à l'eau et on enlève les rouleaux.

Après séchage sous casque, les cheveux présentent de belles boucles.

### EXEMPLE 4 :

On a réalisé les quatre compositions réductrices de déformation permanente suivantes :

### Réducteur 1 (invention) :

| | |
|---|---|
| - acide thioglycolique | 9,2 g |
| - séquestrant | 0,4 g |
| - arginine | 1 g |
| - Mexomère PO | 1 MA g |
| - eau | qsp 100 g |

### Réducteur 2 (invention) :

| | |
|---|---|
| - acide thioglycolique | 9,2 g |
| - séquestrant | 0,4 g |
| - arginine | 1 g |
| - Mexomère PO | 2 MA g |
| - eau | qsp 100 g |

### Réducteur 3 (comparatif) :

| | |
|---|---|
| - acide thioglycolique | 9,2 g |
| - séquestrant | 0,4 g |
| - arginine | 1 g |
| - hydrolysat de collagène N-hydroxypropyl cocoyl diméthyl ammonium en solution aqueuse à 30 % | 1 MA g |
| - eau | qsp 100 g |

### Réducteur 4 (comparatif) :

| | |
|---|---|
| - acide thioglycolique | 9,2 g |
| - séquestrant | 0,4 g |
| - arginine | 1 g |
| - hydrolysat de kératine de laine (PM 1350) quaternisé en solution aqueuse à 30 % | 1 MA g |
| - eau | qsp 100 g |

Chaque composition réductrice a été appliquée sur quatre mèches de cheveux faiblement sensibilisés. Après avoir laissé la composition agir pendant environ 15 minutes, on rince abondamment à l'eau puis on applique la composition oxydante suivante :

| | |
|---|---|
| - eau oxygénée | qsp 8 volumes |
| pH 3 | |

On laisse agir la composition oxydante pendant environ 5 minutes, puis on rince abondamment les mèches à l'eau.

On a ensuite fait subir aux mèches de cheveux un procédé de coloration d'oxydation.

Enfin, on a de nouveau soumis les mèches de cheveux à un procédé de déformation permanente identique à celui décrit ci-dessus.

On a ensuite soumis les mèches de cheveux ainsi traitées au test de solubilité alcaline suivant : on a immergé les cheveux dans une solution de soude de concentration 0,1 mol/l pendant une durée de 30 minutes et à une température de 60 °C. Puis on a pesé la quantité de cheveux restant. On détermine ainsi la quantité de cheveux solubilisés. Cette mesure permet d'évaluer le degré d'altération du cheveu. Plus le cheveu est dégradé, plus il se solubilise.

Les résultats, exprimés en pourcentage de cheveux solubilisés (SA) et ramenés pour chaque composition réductrice à la moyenne des résultats obtenus sur les quatre mèches, sont consignés dans le tableau suivant :

**Tableau**

| Composition | SA |
|---|---|
| Réducteur 1 (invention) | 35,1 % |
| Réducteur 2 (invention) | 32,2 % |
| Réducteur 3 (comparatif) | 49,6 % |
| Réducteur 4 (comparatif) | 53,6 % |

Ces résultats montrent clairement que pour limiter la dégradation mécanique des cheveux soumis à divers traitements, en particulier à des traitements de déformation permanente, les compositions réductrices selon l'invention, à savoir comprenant un polymère cationique comportant des groupements amine primaire, secondaire, tertiaire ou ammonium quaternaire dans la chaîne principale sont plus efficaces que des compositions réductrices comprenant des polymères cationiques ne comportant pas de tels groupements dans la chaîne principale comme les hydrolysats de protéine quaternisés.

## Revendications

1. Composition cosmétique pour le premier temps d'une opération de déformation permanente des matières kératiniques, en particulier des cheveux, consistant à réduire les liaisons disulfures de la kératine, caractérisée par le fait qu'elle comprend au moins un agent actif approprié pour la réduction des liaisons disulfures de la kératine, au moins un agent alcalinisant choisi parmi l'ornithine, la lysine et l'arginine, et au moins un polymère cationique comportant des groupements amine primaire, secondaire, tertiaire ou ammonium quaternaire dans la chaîne principale.

2. Composition selon la revendication précédente, caractérisée en ce que les agents actifs appropriés pour la réduction des liaisons disulfures de la kératine sont des sulfites, des bisulfites ou, de préférence, des thiols, seuls ou en mélange.

3. Composition selon la revendication précédente, caractérisée en ce que les agents actifs appropriés pour la réduction des liaisons disulfures de la kératine sont choisis parmi la cystéïne et ses divers dérivés, la cystéamine et ses dérivés, l'acide 3-mercaptopropionique, l'acide thiolactique, l'acide thioglycolique ainsi que leurs esters ou leurs sels.

4. Composition selon l'une quelconque des revendications précédentes, caractérisée en ce que l'agent alcalinisant est l'arginine.

5. Composition selon l'une quelconque des revendications précédentes, caractérisée en ce que la composition comprend d'autres agents alcalinisants, tels que l'ammoniaque, la monoéthanolamine ou des produits carbonatés.

6. Composition selon la revendication précédente, caractérisée en ce que les autres agents alcalinisants sont présents pour neutraliser les agents réducteurs à caractère acide.

7. Composition selon l'une quelconque des revendications précédentes, caractérisée en ce que les polymères cationiques utilisés dans la présente invention sont choisis parmi :
(1) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères ;
(2) les polyaminoamides solubles dans l'eau, éventuellemnt réticulés et/ou alcoylés ;
(3) les dérivés de polyaminoamides résultant de la condensation de polyalcoylènes polyamines avec des acides polycarboxyliques suivie d'une alcoylation par des agents bifonctionnels ;
(4) les polymères obtenus par réaction d'une polyalkylène polyamine comportant deux groupements amine primaire et au moins un groupement amine secondaire avec un acide dicarboxylique ;
(5) les cyclohomopolymères de méthyl diallyl amine ou de diméthyl diallyl ammonium ;
(6) les polymères de diammonium quaternaire ;
(7) les polymères de polyammonium quaternaire.

8. Composition selon la revendication précédente, caractérisée en ce que les polymères sont choisis parmi le Merquat 100 et le polymère de diammonium quaternaire contenant des motifs récurrants répondant à la formule (III) : 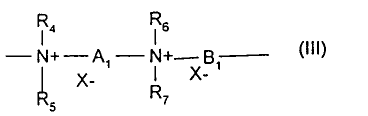 dans laquelle R4, R5, R6 et R7 représentent le radical méthyle, A1 représente le radical de formule -(CH2)3- et B1 représente le radical de formule -(CH2)6- et X⁻représente l'anion chlorure.

9. Composition selon l'une quelconque des revendications précédentes, caractérisée en ce qu'elle comprend un agent tensioactif de type non-ionique, anionique, cationique ou amphotère.

10. Composition selon l'une quelconque des revendications précédentes, caractérisée en ce que l'agent actif approprié pour la réduction des liaisons disulfures de la kératine est associé à au moins un disulfure.

11. Composition selon l'une quelconque des revendications précédentes, caractérisée en ce que l'agent actif approprié pour la réduction des liaisons disulfures de la kératine est présent à une concentration comprise entre 1 et 20 % en poids, et de préférence entre 5 et 15 % en poids, par rapport au poids total de ladite composition.

12. Composition selon l'une quelconque des revendications précédentes, caractérisée en ce que le polymère cationique comportant des groupements amine primaire, secondaire, tertiaire ou ammonium quaternaire dans la chaîne principale est présent à une concentration comprise entre 0,1 et 5 % en poids, et de préférence entre 0,5 et 3 % en poids par rapport au poids total de ladite composition.

13. Composition selon l'une quelconque des revendications précédentes, caractérisée en ce que l'agent alcalinisant est présent à une concentration comprise entre 0,01 et 20 % en poids, et de préférence entre 0,1 et 18 % en poids, par rapport au poids total de la composition réductrice.

14. Composition selon l'une quelconque des revendications précédentes, caractérisée en ce qu'elle se présente sous la forme d'une lotion, épaissie ou non, d'une crème, d'un gel.

15. Dispositif de conditionnement, caractérisé en ce qu'il comprend, dans un ou plusieurs compartiments, une composition définie selon l'une quelconque des revendications précédentes.

16. Dispositif selon la revendication précédente, caractérisé en ce qu'il est à au moins deux compartiments, dans un premier compartiment, se trouve au moins un agent actif approprié pour la réduction des liaisons disulfures de la kératine, et dans un deuxième compartiment, se trouvent au moins un agent alcalinisant choisi parmi l'ornithine, la lysine et l'arginine et événtuellement les parfums.

17. Procédé de traitement des matières kératiniques, en particulier des cheveux, en vue d'obtenir une déformation permanente de ces dernières, en particulier sous la forme de cheveux permanentés, ledit procédé étant caractérisé par le fait qu'il comporte les étapes suivantes : (i) on applique sur la matière kératinique à traiter une composition selon l'une des revendications 1 à 14, les moyens (rouleaux) nécessaires à la mise sous tension mécanique de la matière kératinique étant mis en oeuvre avant, pendant ou après ladite application, (ii) puis on rince la matière kératinique ainsi traitée, (iii) on applique sur la matière kératinique ainsi rincée une composition oxydante, la matière kératinique traitée étant séparée des moyens de mise sous tension utilisés à l'étape (i) avant ou après ladite application de la composition oxydante, (iv) et enfin on rince à nouveau la matière kératinique.

18. Procédé selon la revendication précédente, caractérisé en ce qu'il est mis en oeuvre pour obtenir une chevelure permanentée.

19. Procédé selon l'une des revendications 17 ou 18, caractérisé en ce que l'étape (i) comprend un temps de pose pour laisser agir ladite composition, qui est généralement compris entre 5 et 60 minutes, de préférence entre 5 et 30 minutes.

20. Procédé de défrisage ou de décrêpage des cheveux, caractéresé en ce que l'on applique sur les cheveux une composition selon l'une des revendications 1 à 14, puis l'on soumet les cheveux à une déformation mécanique permettant de les fixer dans leur nouvelle forme, par une opération de lissage des cheveux avec le dos ou les dents d'un peigne ou à la main, pendant le temps de pose de 5 à 60 minutes, en particulier de 5 à 30 minutes, on procède alors à un ou plusieurs nouveaux lissages, puis on rince et on applique une composition oxydante, que l'on laisse agir pendant environ 2 à 10 minutes, puis on rince abondamment les cheveux.

## Claims

1. Cosmetic composition for the first stage of an operation for the permanent reshaping of keratinous material, in particular the hair, which consists in reducing the disulphide bonds of keratin, characterized in that it comprises at least one active agent suitable for the reduction of the disulphide bonds of keratin, at least one basifying agent chosen from ornithine, lysine and arginine, and at least one cationic polymer containing primary, secondary or tertiary amine groups or quaternary ammonium groups in the main chain.

2. Composition according to the preceding claim, characterized in that the active agents suitable for the reduction of the disulphide bonds of keratin are sulphites, bisulphites or, preferably, thiols, alone or as a mixture.

3. Composition according to the preceding claim, characterized in that the active agents suitable for the reduction of the disulphide bonds of keratin are chosen from cysteine and various derivatives thereof, cysteamine and derivatives thereof, 3-mercaptopropionic acid, thiolactic acid and thioglycolic acid, as well as the esters or salts thereof.

4. Composition according to any one of the preceding claims, characterized in that the basifying agent is arginine.

5. Composition according to any one of the preceding claims, characterized in that the composition comprises other basifying agents, such as aqueous ammonia, monoethanolamine or carbonate-based products.

6. Composition according to the preceding claim, characterized in that the other basifying agents are present in order to neutralize the reducing agents of acidic nature.

7. Composition according to any one of the preceding claims, characterized in that the cationic polymers used in the present invention are chosen from:
(1) polymers consisting of piperazinyl units and divalent alkylene or hydroxyalkylene radicals containing straight or branched chains, which are optionally interrupted by oxygen, sulphur or nitrogen atoms or by aromatic or heterocyclic rings, as well as the oxidation and/or quaternization products of these polymers;
(2) water-soluble polyamino amides which are optionally crosslinked and/or alkylated;
(3) polyamino amide derivatives resulting from the condensation of polyalkylene polyamines with polycarboxylic acids followed by an alkylation with difunctional agents;
(4) polymers obtained by reaction of a polyalkylene polyamine containing two primary amine groups and at least one secondary amine group with a dicarboxylic acid;
(5) cyclohomopolymers of methyldiallylamine or of dimethyldiallylammonium;
(6) quaternary diammonium polymers;
(7) quaternary polyammonium polymers.

8. Composition according to the preceding claim, characterized in that the polymers are chosen from Merquat 100 and the quaternary diammonium polymer containing repeating units corresponding to the formula (III): 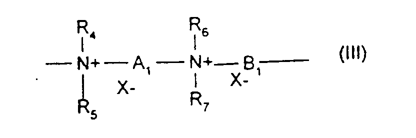 in which R4, R5, R6 and R7 represent a methyl radical, A1 represents a radical of formula -(CH2)3- and B1 represents a radical of formula -(CH2)6- and X⁻ represents a chloride anion.

9. Composition according to any one of the preceding claims, characterized in that it comprises a surfactant of nonionic, anionic, cationic or amphoteric type.

10. Composition according to any one of the preceding claims, characterized in that the active agent suitable for the reduction of the disulphide bonds of keratin is combined with at least one disulphide.

11. Composition according to any one of the preceding claims, characterized in that the active agent suitable for the reduction of the disulphide bonds of keratin is present at a concentration between 1 and 20 % by weight and preferably between 5 and 15 % by weight relative to the total weight of the said composition.

12. Composition according to any one of the preceding claims, characterized in that the cationic polymer containing primary, secondary or tertiary amine groups or quaternary ammonium groups in the main chain is present at a concentration between 0.1 and 5 % by weight and preferably between 0.5 and 3 % by weight relative to the total weight of the said composition.

13. Composition according to any one of the preceding claims, characterized in that the basifying agent is present at a concentration between 0.01 and 20 % by weight, and preferably between 0.1 and 18 % by weight relative to the total weight of the reducing composition.

14. Composition according to any one of the preceding claims, characterized in that it is in the form of a lotion, which may or may not be thickened, a cream or a gel.

15. Packaging device, characterized in that it comprises, in one or more compartments, a composition defined according to any one of the preceding claims.

16. Device according to the preceding claim, characterized in that it contains at least two compartments; at least one active agent which is suitable for the reduction of the disulphide bonds of keratin is found in a first compartment, and at least one basifying agent chosen from ornithine, lysine and arginine and optionally the fragrances are found in the second compartment.

17. Process for the treatment of keratinous material, in particular the hair, for the purpose of obtaining a permanent reshaping of the latter, in particular in the form of permanent-waved hair, the said process being characterized in that it includes the following steps: (i) a composition according to one of Claims 1 to 14 is applied to the keratinous material to be treated, the means (rollers) necessary for placing the keratinous material under mechanical tension being implemented before, during or after the said application, (ii) the keratinous material thus treated is then rinsed, (iii) an oxidizing composition is applied to the keratinous material thus rinsed, the treated keratinous material being separated from the means for placing under tension used in step (i) before or after the said application of the oxidizing composition, (iv) and, lastly, the keratinous material is rinsed again.

18. Process according to the preceding claim, characterized in that it is carried out in order to obtain a head of permanent-waved hair.

19. Process according to either of Claims 17 and 18, characterized in that the step (i) comprises an exposure time, in order to allow the said composition to act, which is generally between 5 and 60 minutes, preferably between 5 and 30 minutes.

20. Process for straightening or removing the curliness from the hair, characterized in that a composition according to one of Claims 1 to 14 is applied to the hair and the hair is then subjected to a mechanical reshaping which allows it to be fixed in its new shape, by an operation of smoothing out the hair with the back or the teeth of a comb or by hand, during the exposure time of 5 to 60 minutes, in particular of 5 to 30 minutes, the hair is again smoothed out one or more times and is then rinsed thoroughly and an oxidizing composition is applied, which is left to act for about 2 to 10 minutes, then the hair is rinsed thoroughly.

## Patentansprüche

1. Kosmetische Zusammensetzung für den ersten Schritt einer permanenten Verformung von Keratinmaterialien und insbesondere des Haares, der darin besteht, die Disulfidbindungen des Keratins zu reduzieren, dadurch gekennzeichnet, daß sie mindestens einen Wirkstoff, der für die Reduktion der Disulfidbindungen des Keratins geeignet ist, mindestens ein Alkalisierungsmittel, das unter Ornithin, Lysin und Arginin ausgewählt ist, und mindestens ein kationisches Polymer enthält, welches primäre, sekundäre, tertiäre oder quaternäre Aminogruppen in der Hauptkette aufweist.

2. Zusammensetzung nach dem vorhergehenden Anspruch, dadurch gekennzeichnet, daß die Wirkstoffe, die für die Reduktion der Disulfidbindungen des Keratins geeignet sind, Sulfite, Hydrogensulfite oder vorzugsweise Thiole oder deren Gemische sind.

3. Zusammensetzung nach dem vorhergehenden Anspruch, dadurch gekennzeichnet, daß die Wirkstoffe, die für die Reduktion der Disulfidbindungen des Keratins geeignet sind, unter Cystein und seinen verschiedenen Derivaten, Cysteamin und seinen Derivaten, 3-Mercaptopropionsäure, Thiomilchsäure, Thioglykolsäure sowie deren Estern oder Salzen ausgewählt sind.

4. Zusammensetzung nach einem der vorhergehenden Ansprüche, dadurch gekennzeichnet, daß das Alkalisierungsmittel Arginin ist.

5. Zusammensetzung nach einem der vorhergehenden Ansprüche, dadurch gekennzeichnet, daß die Zusammensetzung weitere Alkalisierungsmittel, wie Ammoniak, Monoethanolamin oder carbonathaltige Produkte, enthält.

6. Zusammensetzung nach dem vorhergehenden Anspruch, dadurch gekennzeichnet, daß die weiteren Alkalisierungsmittel vorliegen, um die Reduktionsmittel mit saurer Charakter zu neutralisieren.

7. Zusammensetzung nach einem der vorhergehenden Ansprüche, dadurch gekennzeichnet, daß die erfindungsgemäß verwendeten kationischen Polymere ausgewählt sind unter:
(1) Polymeren, die aus Piperazinyleinheiten und zweiwertigen Alkylen- oder Hydroxyalkylengruppen mit geraden oder verzweigten Ketten, welche gegebenenfalls durch Sauerstoffatome, Schwefelatome, Stickstoffatome oder aromatische oder heterocyclische Ringe unterbrochen sind, bestehen, sowie den Oxidationsprodukten und/oder Quaternisierungsprodukten dieser Polymere;
(2) wasserlöslichen Polyaminoamiden, die gegebenenfalls vernetzt und/oder alkyliert sind;
(3) Polyaminoamidderivaten, die aus der Kondensation von Polyalkylenpolyaminen mit Polycarbonsäuren und nachfolgender Alkylierung mit bifunktionellen Mitteln resultieren;
(4) Polymeren, die durch die Umsetzung eines Polyalkylenpolyamins mit zwei primären Aminogruppen und mindestens einer sekundären Aminogruppe mit einer Dicarbonsäure hergestellt sind;
(5) Dimethyldiallylamin- oder Dimethyldiallylammonium-Cyclohomopolymeren;
(6) quaternären Diammoniumpolymeren;
(7) quaternären Polyammoniumpolymeren;

8. Zusammensetzung nach einem der vorhergehenden Ansprüche, dadurch gekennzeichnet, daß die Polymere unter Merquat 100 und dem quaternären Diammoniumpolymer mit wiederkehrenden Einheiten der Formel (III) ausgewählt sind: 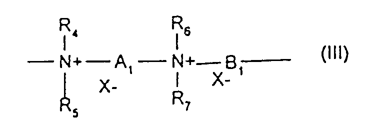 worin R4, R5, R6 und R7 Methyl, A1 die Gruppe der Formel -(CH₂)₃- und B1 die Gruppe der Formel -(CH₂)₆- und X⁻ das Chloridanion bedeuten.

9. Zusammensetzung nach einem der vorhergehenden Ansprüche, dadurch gekennzeichnet, daß sie ein grenzflächenaktives Mittel vom nichtionischen, anionischen, kationischen oder amphoteren Typ enthält.

10. Zusammensetzung nach einem der vorhergehenden Ansprüche, dadurch gekennzeichnet, daß der Wirkstoff, der für die Reduktion der Disulfidbindungen des Keratins geeignet ist, mit mindestens einem Disulfid kombiniert ist.

11. Zusammensetzung nach einem der vorhergehenden Ansprüche, dadurch gekennzeichnet, daß der Wirkstoff, der für die Reduktion der Disulfidbindungen des Keratins geeignet ist, in einer Konzentration im Bereich von 1 bis 20 Gew.-% und vorzugsweise von 5 bis 16 Gew.-%, bezogen auf das Gesamtgewicht der Zusammensetzung, vorliegt.

12. Zusammensetzung nach einem der vorhergehenden Ansprüche, dadurch gekennzeichnet, daß das kationische Polymer, das primäre, sekundäre, tertiäre oder quaternäre Aminogruppen in der Hauptkette aufweist, in einer Konzentration von 0,1 bis 5 Gew.-% und vorzugsweise von 0,5 bis 3 Gew.-%, bezogen auf das Gesamtgewicht der Zusammensetzung, vorliegt.

13. Zusammensetzung nach einem der vorhergehenden Ansprüche, dadurch gekennzeichnet, daß das Alkalisierungsmittel in einer Konzentration von 0,01 bis 20 Gew.-% und vorzugsweise von 0,1 bis 18 Gew.-%, bezogen auf das Gesamtgewicht der reduzierenden Zusammensetzung, vorliegt.

14. Zusammensetzung nach einem der vorhergehenden Ansprüche, dadurch gekennzeichnet, daß die Zusammensetzung in Form einer gegebenenfalls dickflüssigen Lotion, einer Creme oder eines Gels vorliegt.

15. Vorrichtung zur Konfektionirung, dadurch gekennzeichnet, daß sie in einer oder mehreren Abteilungen eine Zusammensetzung nach einem der vorhergehenden Ansprüche enthält.

16. Vorrichtung nach dem vorhergehenden Anspruch, dadurch gekennzeichnet, daß sie mindestens zwei Abteilungen aufweist, wobei in einer ersten Abteilung mindestens ein Wirkstoff, der für die Reduktion der Disulfidbindungen des Keratins geeignet ist, und in einer zweiten Abteilung mindestens ein Alkalisierungsmittel, das unter Ornithin, Lysin und Arginin ausgewählt ist, und gegebenenfalls die Parfums vorliegen.

17. Verfahren zur Behandlung von Keratinmaterialien, insbesondere des Haares, zur Erzielung einer permanenten Verformung des Haares, insbesondere in Form von dauergewelltem Haar, wobei das Verfahren dadurch gekennzeichnet ist, daß es die folgenden Schritte umfaßt: (i) Auftragen einer Zusammensetzung nach einem der Ansprüche 1 bis 14 auf das zu behandelnde Keratinmaterial, wobei die Mittel (Rollen), die erforderlich sind, um das Keratinmaterial unter mechanische Spannung zu setzen, vor, während oder nach dem Auftragen angebracht werden, (ii) Spülen des so behandelten Keratinmaterials, (iii) Auftragen einer oxidierenden Zusammensetzung auf das so gespülte Keratinmaterial, wobei das behandelte Keratinmaterial von den in Schritt (i) verwendeten Mitteln, mit denen es unter Spannung gesetzt wird, vor oder nach dem Auftragen der oxidierenden Zusammensetzung getrennt wird, und (iv) schließlich nochmaliges Spülen des Keratinmaterials.

18. Verfahren nach dem vorhergehenden Anspruch, dadurch gekennzeichnet, daß es zur Erzielung von dauergewelltem Haar angewandt wird.

19. Verfahren nach einem der Ansprüche 17 oder 18, dadurch gekennzeichnet, daß Schritt (i) eine Einwirkungszeit umfaßt, um die Zusammensetzung wirken zu lassen, die im allgemeinem im Bereich von 5 bis 60 Minuten und vorzugsweise von 5 bis 30 Minuten liegt.

20. Verfahren zum Entfernen einer Wellung oder zur Entkräuselung des Haares, dadurch gekennzeichnet, daß auf das Haar eine Zusammensetzung nach einem der Ansprüche 1 bis 14 aufgetragen wird, worauf das Haar mechanisch durch eine Glättung des Haares mit den Fingern oder den Zähnen eines Kammes oder der Hand verformt wird, wodurch es während einer Einwirkungszeit von 5 bis 60 Minuten und insbesondere 5 bis 30 Minuten in der neuen Form fixiert wird, worauf eine oder mehrere nochmalige Glättungen durchgeführt, gespült und eine oxidierende Zusammensetzung aufgetragen wird, die etwa 2 bis 10 Minuten einwirken gelassen wird, worauf das Haar ausgiebig gespült wird.
